# EUROPÄISCHE PATENTSCHRIFT

(11) **EP 3 849 632 B1**
(45) Veröffentlichungstag und Bekanntmachung des Hinweises auf die Patenterteilung: **27.03.2024**
(21) Anmeldenummer: 19769787.3
(22) Anmeldetag: 16.09.2019
(51) Int. Cl.: A61M 1/16, A61M 1/34, A61M 1/36

(54) **EXTRAKORPORALE BLUTBEHANDLUNGSVORRICHTUNG**
DEVICE FOR EXTRACORPOREAL TREATMENT OF BLOOD
DISPOSITIF POUR LE TRAITEMENT EXTRACORPOREL DE SANG

(30) Priorität: 14.09.2018 DE 102018122583
(43) Veröffentlichungstag der Anmeldung: 21.07.2021
(73) Patentinhaber: Fresenius Medical Care Deutschland GmbH, 61352 Bad Homburg (DE)
(72) Erfinder: OFFERMANNS, Lars, 35510 Butzbach (DE)
(74) Vertreter: Nordmeyer, Philipp Werner
(86) Internationale Anmeldenummer: PCT/EP2019/074661
(87) Internationale Veröffentlichungsnummer: WO 2020/053442

(56) Entgegenhaltungen:
- DE-A1-102007 024 463
- US-B1- 6 468 424

## Beschreibung

### Technisches Gebiet

Die vorliegende Erfindung betrifft eine extrakorporale Blutbehandlungsvorrichtung und ein Verfahren zum Überwachen einer Anschlussstelle einer extrakorporalen Blutbehandlungsvorrichtung.

### Stand der Technik

Aus dem Stand der Technik sind extrakorporale Blutbehandlungsvorrichtungen zum Behandeln von Patienten bekannt, beispielsweise zum Durchführen einer Dialyse. Die extrakorporalen Blutbehandlungsvorrichtungen umfassen dabei üblicher Weise Fluidsysteme, durch welche hindurch sowohl das Blut des Patienten, als auch weitere, für die Behandlung des Patienten relevante Stoffe fließen können. Die Fluidsysteme können beispielsweise in Form von Schlauchsystemen ausgebildet sein, die sowohl das Patientenblut als auch Dialyseflüssigkeit transportieren können. Mit diesen Fluidsystemen können medizinische Flüssigkeiten dem Patienten zugeführt und/oder Blut dem Patienten entnommen werden. Solche Schlauchsysteme bilden insbesondere in Verbindung mit der entsprechenden Behandlungsvorrichtung komplexe Systeme aus.

Solche Fluidsysteme und insbesondere Schlauchsets weisen häufig Anschlussstellen auf, welche das Zugeben beziehungsweise Verabreichen von zusätzlichen Stoffen, wie beispielsweise Medikamenten oder sonstigen Infusionslösungen, während einer Behandlung ermöglichen. Solche Anschlussstellen sind in der Regel im regulären Betrieb oder zumindest zu Beginn der Behandlung geschlossen, können jedoch optional genutzt werden. Dazu werden an die Anschlussstellen beispielsweise Spritzen oder Beutel oder weitere Leitungen, die mit einer Zugabevorrichtung, wie beispielsweise einer Spritzenpumpe, versehen sind, angeschlossen, mittels denen der jeweilige Stoff dann über die Anschlussstelle dem Patienten verabreicht werden kann,

Problematisch ist hierbei, dass insbesondere in komplexeren Systemen das Risiko besteht, dass ein Benutzer nicht die richtige, für den beabsichtigten Zweck geeignete Anschlussstelle, sondern versehentlich eine an einer anderen Stelle angeordnete Anschlussstelle verwendet und somit den Patienten potenziell gefährden kann. Beispielsweise kann ein Medikament vollständig und in kürzester Zeit in die Blutbahn des Patienten gelangen, anstatt kontinuierlich zugegeben zu werden. Ebenfalls kann dies dazu führen, dass ein Medikament im falschen Fluidsystem verabreicht wird. Beispielsweise kann eine Spülflüssigkeit statt in den Dialysatkreislauf in den Blutkreislauf des Patienten verabreicht werden oder ein Medikament überhaupt nicht in die Blutbahn des Patienten gelangen, da es stattdessen dem Dialysatkreislauf zugeführt wird.

Zum Überprüfen eines korrekten Flussverlaufs in einfachen Infusionssystemen ist beispielsweise aus der WO 2013/138537 A1 ein entsprechendes Messverfahren bekannt, welches bei einer Fehlfunktion optische oder akustische Signale ausgibt, wobei anhand der Flussmessung weiterhin eine nachfolgende Regelung des Flusses möglich ist.

Ebenfalls sind beispielsweise aus der EP 2 150 293 A1 Zugabevorrichtungen bekannt, welche eine korrekte Ankopplung mittels einer Druckmessung überprüfen. Eine Überwachung der korrekten Funktion einer Zugabevorrichtung mittels einer Druckmessung ist weiterhin auch aus der DE 10 207 044 413 A1 bekannt.

Durch die Überprüfung der korrekten Funktion beziehungsweise der korrekten Flussgeschwindigkeit wird jedoch nicht überprüft, ob die Zugabe eines Stoffs an einer dafür vorgesehenen Stelle erfolgt, sodass diesbezüglich weitere Verbesserungen erforderlich sind, um die Sicherheit des Patienten zu erhöhen.

Aus der DE 10 2007 024 463 A1 ist eine Vorrichtung zur Überprüfung der korrekten Ankopplung einer Zugabevorrichtung an ein Therapiegerät bekannt, wobei mittels eines Drucksensors überprüft werden kann, ob eine Ankopplung korrekt ausgeführt ist.

Weiterhin offenbart die US 6 468 424 B1 eine Dialysemaschine mit einer Detektionsanordnung, die dazu konfiguriert ist, einen zuzugebenden Stoff mittels eines Identifizierungsmittels auf dem Konnektor des Stoffbehälters zu überprüfen und ein Warnsignal auszugeben, wenn der Stoffbehälter nicht der richtige ist.

### Darstellung der Erfindung

Ausgehend von dem bekannten Stand der Technik ist es eine Aufgabe der vorliegenden Erfindung, eine verbesserte Vorrichtung zum Überwachen von Anschlussstellen sowie ein entsprechendes Verfahren bereitzustellen.

Die Aufgabe wird durch eine extrakorporale Blutbehandlungsvorrichtung mit den Merkmalen des Anspruchs 1 gelöst. Vorteilhafte Weiterbildungen ergeben sich aus den Unteransprüchen. Entsprechend wird eine extrakorporale Blutbehandlungsvorrichtung vorgeschlagen, umfassend ein Fluidsystem mit mindestens einer Anschlussstelle zum Zugeben von Stoffen und mindestens einer Detektionsanordnung zum Detektieren eines Stoffflusses an der Anschlussstelle, wobei die Detektionsanordnung dazu eingerichtet ist, ein Flusssignal zu erzeugen, wenn ein Stofffluss an der Anschlussstelle detektiert wird, und eine Steuereinheit, welche mit der Detektionsanordnung kommunizierend verbunden ist und dazu eingerichtet ist, in Reaktion auf den Empfang eines Flusssignals ein Signal auszugeben.

Das Ausgeben eines Signals basierend auf dem detektierten Stofffluss hat den Vorteil, dass ein Benutzer der extrakorporalen Blutbehandlungsvorrichtung bei einer vorgenommenen Zugabe darauf aufmerksam gemacht wird, dass an der entsprechenden Anschlussstelle ein Stoff zugegeben wird. Entsprechend kann beispielsweise eine gegebenenfalls versehentliche Zugabe des Stoffs verhindert und/oder sichergestellt werden, dass die Zugabe an sich in Ordnung ist und dass sie an einer geeigneten Stelle des Fluidsystems erfolgt.

Mit anderen Worten kann die Zugabe eines Stoffs an einer Anschlussstelle überwacht werden, sodass das Auftreten von eventuell patientenschädigenden Situationen vermieden und gleichzeitig eine erwünschte therapeutische Wirkung garantiert werden kann. Dies ist insbesondere vorteilhaft bei einer Zugabe von Stoffen an Anschlussstellen, welche unmittelbar mit dem extrakorporalen Blutkreislauf eines Patienten verbunden sind, zumal ein direktes Infundieren eines Stoffs in die Blutbahn des Patienten ein erhöhtes Risiko beziehungsweise eine Gefährdung des Patienten darstellen kann, wenn es nicht erwünscht ist.

Durch Ausgabe des Signals wird der Benutzer oder Bediener der Anschlussstelle der extrakorporalen Blutbehandlungsvorrichtung, beispielsweise medizinisches Personal, sofort auf die jeweilige Verwendung aufmerksam gemacht und somit bei der Bedienung oder Aufrüstung der extrakorporalen Blutbehandlungsvorrichtung unterstützt. Beispielsweise kann somit sichergestellt werden, dass der Stoff an der richtigen Anschlussstelle im Fluidsystem und/oder an eine vorgesehene Pumpe angeschlossen wird, so dass beispielsweise eine vorgesehene Pumpenrate beziehungsweise Förderrate eingehalten und/oder der Patient mit der vorgesehenen Dosis eines Medikaments versorgt wird. Das Ausgeben des Signals kann entsprechend sowohl während des Betriebs als auch in angehaltenem beziehungsweise inaktivem Zustand der extrakorporalen Blutbehandlungsvorrichtung erfolgen.

Als extrakorporale Blutbehandlungsvorrichtung sind verschiedene Vorrichtungen bekannt, welche einem Patienten Blut beispielsweise über einen arteriellen Zugang entnehmen, das entnommene Blut verarbeiten, beispielsweise reinigen, auftrennen und/oder separieren, umleiten, oxygenieren, entsäuern, und/oder dialysieren, und anschließend dem Patienten beispielsweise über einen venösen Zugang wieder zurückführen. Das Fluidsystem kann in der einfachsten Ausführung beispielsweise einen einfachen Schlauch oder eine Schlauchverbindung darstellen, kann jedoch auch ein Schlauchsystem umfassen, wobei verschiedene Abzweigungen und/oder Schlauchverbindungen vorgesehen sein können. Beispielsweise kann das Schutzsystem einen Kreislauf bilden, wobei eine oder mehrere Anschlussstellen an dem Kreislauf vorgesehen sind. Alternativ oder zusätzlich kann das Fluidsystem ebenfalls Reservoirs, Behälter, Beutel und/oder Pumpen aufweisen oder lediglich daraus ausgebildet sein. Entsprechend kann eine Anschlussstelle als Fluidkupplung vorgesehen sein, beispielsweise als Luer-Lock Verbindung oder selbstschließende Membran, sowohl unmittelbar als auch mittelbar, beispielsweise mit einer Schlauchverbindung.

Als Stoffe, welche zugegeben werden können, werden grundsätzlich alle Stoffe verstanden, welche eine mittelbare oder unmittelbare therapeutische Wirkung bei der vorgenommenen oder vorzunehmenden Behandlung des Patienten bewirken. So können die Stoffe medizinische Flüssigkeiten, beispielsweise Medikamente wie zum Beispiel Blutdruckstabilisatoren, Entzündungshemmer, oder Schmerzmittel, Substitutionslösungen, wie zum Beispiel Salzlösungen, und/oder Flüssigkeiten für therapeutische Nebensymptome wie zum Beispiel Glukose, Heparin, oder Insulin, umfassen. Beispiele für Flüssigkeiten mit mittelbarer therapeutischer Wirkung können ebenfalls Substitutionslösungen wie zum Beispiel Salzlösungen oder Glukose umfassen, können aber auch Dialysat oder, in einfachster Form, Wasser darstellen.

Die Detektionsanordnung kann aus einer oder mehreren Einheiten gebildet sein, wobei die mindestens eine Einheit entweder für eine Anschlussstelle oder für eine Mehrzahl von Anschlussstellen vorgesehen ist. Beispielsweise kann eine Einheit derart ausgebildet sein, dass diese den Stofffluss von zwei benachbarten Anschlüssen detektiert beziehungsweise bestimmt und den jeweiligen Stofffluss über ein entsprechendes Flusssignal an die Steuereinheit weitergibt. Ebenfalls kann eine Mehrzahl von Einheiten vorgesehen sein, wobei jede Einheit den Stofffluss einer jeweiligen Anschlussstelle bestimmt und über eine entsprechende Schaltung entweder unmittelbar oder über eine zentrale Detektionseinheit an die Steuereinheit weiterleitet.

Durch das Detektieren eines Stoffflusses kann eine Verwendung beziehungsweise Betätigung der Anschlussstelle erfasst werden. Entsprechend kann bestimmt werden, ob die Anschlussstelle aktiv oder inaktiv ist. Bevorzugt ermöglicht der detektierte Stofffluss, dass eine Aussage über die jeweilige Verwendung getroffen werden kann, beispielsweise über die Art, Dauer oder den Umfang der Verwendung.

Um ein dem detektierten Stofffluss entsprechendes Signal auszugeben, ist weiterhin eine Steuereinheit vorgesehen, welche beispielsweise eine logische Ebene für die Verarbeitung von Flusssignalen darstellt. Beispielsweise kann die Steuereinheit einen Prozessor beziehungsweise Mikroprozessor und/oder eine entsprechende Schaltung aufweisen, welche(r) die empfangenen Flusssignale auswertet beziehungsweise verarbeitet und anhand dessen das Ausgeben eines Signals durch eine entsprechende Steuerung/Regelung veranlassen kann. Das Flusssignal kann somit eine Information beziehungsweise ein Signal sein, welche(s) mit dem detektierten Stofffluss übereinstimmt. Je nach Ausgestaltung der Detektionsanordnung und/oder des Fluidsystems beziehungsweise der einen oder mehreren Anschlussstellen oder generell der mit der erforderlichen Überwachung der Anschlussstellen einhergehenden Komplexität, kann die Steuereinheit von der Detektionsanordnung getrennt oder mit dieser als Einheit kombiniert sein.

Um einen Bediener oder Benutzer auf die an der Anschlussstelle vorgenommene Handlung aufmerksam zu machen beziehungsweise darauf hinzuweisen, dass ein Stofffluss im Begriffe ist, gestartet zu werden, ist das Signal bevorzugt ein akustisches, mechanisches und/oder optisches Signal, sodass dies in der einfachsten Form das Ausgeben eines Tons, einer Vibration beziehungsweise das Aufleuchten einer Leuchte umfassen kann. Bevorzugt umfasst das Signal jedoch genauere Informationen über den Stofffluss und/oder einer gegebenenfalls vorzunehmenden Handlung. Beispielsweise kann der Ton eine Sprachnachricht aufweisen, welche den Stofffluss an der Anschlussstelle bestätigt und/oder eine Aufforderung für den Benutzer darstellt. Somit kann durch das Signal beispielsweise auf eine erforderliche Überprüfung und/oder einen aktiven oder inaktiven Status der Anschlussstelle oder der extrakorporalen Blutbehandlungsvorrichtung hingewiesen werden. Das Signal, insbesondere ein optisches Signal, kann entweder von der Steuereinheit oder von der Anschlussstelle beziehungsweise Detektionsanordnung ausgehen, kann jedoch auch dezentral beziehungsweise an einer anderen Stelle der Blutbehandlungsvorrichtung vorgesehen sein.

Bevorzugt umfasst das Fluidsystem mindestens zwei Anschlussstellen zum Zugeben von Stoffen, wobei die Steuereinheit dazu eingerichtet ist, ein Flusssignal einer der Anschlussstellen zuzuordnen und das Signal entsprechend der zugeordneten Anschlussstelle auszugeben.

Dadurch, dass mehrere Anschlussstellen vorgesehen sind und die Steuereinheit den Stofffluss einer spezifischen Anschlussstelle zuordnen kann, ist eine Überwachung der Anschlussstellen beziehungsweise der Zugabe von Stoffen auch in einem komplexeren System möglich. Beispielsweise kann die Steuervorrichtung ein Signal ausgeben, welches für die jeweilige Anschlussstelle oder zumindest für ein Subsystem der Blutbehandlungsvorrichtung beziehungsweise des Fluidsystems spezifisch ist. Somit wird dem Benutzer durch das Signal eine Rückkopplung beziehungsweise ein Feedback gegeben, welche(s) die verwendete Anschlussstelle bestätigt und/oder eine zu verwendende Anschlussstelle angibt. Der Benutzer wird somit an einem angehenden Fehler gehindert, sodass die Sicherheit des Patienten weiter erhöht wird, ohne dass es dabei kognitive Anstrengungen des Benutzers erfordert.

Weiterhin ist die Steuereinheit bevorzugt dazu eingerichtet, das Signal basierend auf einem Vergleich des detektierten Stoffflusses mit einem für die Anschlussstelle vorgegebenen Flusszustand auszugeben.

In der einfachsten Ausführung beziehungsweise mit nur einer Anschlussstelle kann dies bereits den Vorteil haben, dass diese Anschlussstelle nicht verwendet wird, da ein entsprechender Flusszustand während des Betriebs der extrakorporalen Blutbehandlungsvorrichtung nicht vorgesehen ist. Zum Beispiel kann eine Anschlussstelle im Fluidsystem stromaufwärts der extrakorporalen Blutbehandlungsvorrichtung, zum Beispiel an einem Schlauch, welcher einen arteriellen Zugang des Patienten mit der Blutbehandlungsvorrichtung verbindet, angeordnet sein und wird nur vor der Behandlung oder beim Anhalten der Behandlung verwendet, beispielsweise zum Kalibrieren oder Reinigen der Blutbehandlungsvorrichtung. Sollte der Benutzer eine Zugabe eines Stoffs an dieser Anschlussstelle vornehmen und damit einen Stofffluss verursachen, so weicht dieser von dem vorgegebenen Flusszustand ab und es wird entsprechend ein Signal ausgegeben.

Insbesondere wenn mehrere Anschlussstellen vorgesehen sind, hat dies den Vorteil, dass für jede der Anschlussstellen ein Flusszustand vorgesehen sein kann, welcher nicht nur auf die jeweilige Verwendung schließen lässt, sondern optional ebenfalls einen zuzugebenden Stoff umfasst. Mit anderen Worten kann für eine bestimmte Anschlussstelle beispielsweise die Zugabe eines bestimmten Medikaments vorgesehen sein, sodass das ausgegebene Signal eine Information bezüglich des entsprechenden Medikaments enthält.

Beispielsweise kann das Signal als Rückkopplung einen Ton enthalten, welcher das vorgesehene Medikament in gesprochener Form enthält. Der Benutzer wird somit entweder darin bestätigt, dass der richtige Stoff zugegeben wird oder enthält eine entsprechende Warnung, dass die vorgenommene Zugabe nicht dem vorgesehenen Stoff entspricht. Alternativ oder zusätzlich kann die Rückkopplung ebenfalls einen Hinweis auf eine eingestellte Förderrate beziehungsweise Pumpenrate enthalten.

Bevorzugt ist die Detektionsanordnung derart ausgebildet, dass diese für jede Anschlussstelle jeweils einen Drucksensor und/oder einen Durchflusssensor umfasst, wobei das Detektieren des Stoffflusses das Erfassen eines Drucks und/oder eines Flusses umfasst.

Entsprechend kann die Detektionsanordnung dazu eingerichtet sein, einen aktuellen Sensorwert und/oder einen Verlauf des Sensorwerts zu ermitteln beziehungsweise zu erfassen, wobei der Stofffluss anhand des Sensorwerts bestimmt wird. Beispielsweise kann der Sensorwert über einen bestimmten Zeitraum variieren oder oszillieren, sodass eine Messung dieses Werts eine genauere Darstellung der erfassten Druck- oder Flussänderung wiedergibt und entsprechend genauer bestimmt werden kann. Mit anderen Worten kann ein Vergleich über einen bestimmten Zeitraum geringfügige Fluktuationen unberücksichtigt lassen und gleichzeitig einen genaueren Verlauf ermöglichen, sodass der detektierte Stofffluss beispielsweise einer bestimmten, vorgenommenen Handlung zugeordnet werden kann. Insbesondere kann auf diese Weise zwischen verschiedenen Ankopplungen oder Betriebsmodi unterschieden werden, sodass Unregelmäßigkeiten oder unerwünschte Situationen, beispielsweise ein beeinträchtigter Fluss oder eine versehentliche Entkopplung an einer Anschlussstelle bestimmt werden können.

Bevorzugt ist ein Display vorgesehen und das von der Steuereinheit ausgegebene Signal umfasst die Ausgabe eines Hinweises auf dem Display.

Als Display können einer oder mehrere Monitore oder sonstige Anzeigemittel vorgesehen sein, welche dazu geeignet sind, den Hinweis grafisch darzustellen. Beispielsweise kann das Display in Form eines Touchpads oder auch eines zentralen Monitors der extrakorporalen Blutbehandlungsvorrichtung vorgesehen sein. Weiterhin kann das Display als mobiles Display vorgesehen sein, beispielsweise als Mobilgerät, PDA, Tablet, Notebook beziehungsweise Laptop, oder als Smartwatch, wobei das mobile Display kommunikativ mit der Steuervorrichtung drahtlos verbunden ist, beispielsweise über WLAN, Bluetooth, Zigbee, NFC, Infrarot, RFID, oder dergleichen.

Der Hinweis umfasst dabei bevorzugt eine grafische Darstellung des Stoffflusses, der jeweiligen Anschlussstelle, der vorgesehenen Anschlussstelle, des vorgesehenen Stoffs, des Behandlungsverlaufs anhand eines erfassten Parameters, und/oder eines Warnsignals, je nach Ausgestaltung der extrakorporalen Blutbehandlungsvorrichtung. Entsprechend wird dem Benutzer ein visuelles Feedback gegeben, sodass eine eventuell versehentliche Verwendung rückgängig gemacht oder verhindert werden kann und gleichzeitig eine korrekte Behandlung des Patienten sichergestellt wird.

Um die Sicherheit des Patienten weiter zu erhöhen, wird die jeweilige Anschlussstelle für die Zugabe eines Stoffs bevorzugt erst nach einer Benutzerbestätigung freigeschaltet. Entsprechend weist mindestens eine der Anschlussstellen ein Verschlusselement, bevorzugt eine Klemme, auf, wobei jedes der Verschlusselemente mit der Steuereinheit kommunikativ verbunden ist und wobei die Steuereinheit dazu eingerichtet ist, eine Benutzeraufforderung in Reaktion auf das Vorliegen des Signals auszugeben und das Verschlusselement basierend auf einer Benutzereingabe zu betätigen oder zumindest teilweise zu öffnen und/oder basierend auf einer Benutzereingabe ein weiteres Signal auszugeben.

Beispielsweise kann an jeder Anschlussstelle eine Klemme vorgesehen sein, welche sich zunächst in einem geschlossenen Zustand befindet. Wenn der Benutzer anschließend die Anschlussstelle betätigt beziehungsweise diese aktiviert oder eine Fluidkopplung bereitstellt, so wird dies entsprechend über den detektierten Stofffluss erfasst und es wird durch die Steuereinheit beispielsweise nicht nur ein Hinweis, sondern auch eine Benutzeraufforderung als Signal ausgegeben. In einer einfachen Form umfasst die Benutzereingabe lediglich eine Bestätigung beziehungsweise eine Lesebestätigung oder ein Einverständnis mit dem Hinweis, wobei die Klemme erst nach einer entsprechenden Eingabe des Benutzers geöffnet wird.

Es kann aber auch vorgesehen sein, dass die Benutzeraufforderung das Eingeben von weiteren Daten erfordert, beispielsweise das Eingeben des entsprechenden Stoffs für die Zugabe in das Fluidsystem. Obwohl es vorgesehen sein kann, dass die Klemme nach einer solchen Eingabe ebenfalls geöffnet wird, wird die Eingabe bevorzugt zunächst mit einem für die Anschlussstelle vorgegebenen Flusszustand - beispielsweise auch für den eingegebenen Stoff - verglichen, sodass die Klemme je nach Bewertung geöffnet wird oder geschlossen bleibt.

Entsprechend kann die Steuereinheit weiterhin dazu eingerichtet sein, das Verschlusselement bei Vorliegen des Flusssignals nach einem Vergleich mit einem für die Anschlussstelle vorgegebenen Flusszustand zu betätigen oder zumindest teilweise zu öffnen.

Eine solche Abhängigkeit des Zustands des jeweiligen Verschlusselements ist besonders vorteilhaft, wenn mehrere Anschlussstellen vorgesehen sind und eine entsprechende Zuordnung des Stoffflusses zu einer spezifischen Anschlussstelle erfolgt. Hier kann die Steuereinheit auch bei komplexeren Systemen sicherstellen, dass der Benutzer dem Patienten nicht versehentlich Flüssigkeiten verabreicht, welche nicht oder zumindest nicht an der jeweiligen Anschlussstelle vorgesehen sind. Gleichzeitig kann der Benutzer durch entsprechende Hinweise auf die vorgesehene Anschlussstelle aufmerksam gemacht werden, sodass die erforderliche therapeutische Wirkung sichergestellt und die Bedienung der extrakorporalen Blutbehandlungsvorrichtung vereinfacht wird.

Bevorzugt ist die Steuereinheit weiterhin dazu eingerichtet, bei Vorliegen des Flusssignals für die Anschlussstelle eine Flussrate, basierend auf einem Vergleich einer vorliegenden Flussrate mit einer für die Anschlussstelle vorgegebenen Flussrate und/oder basierend auf einer Benutzereingabe einzustellen.

Dies hat den Vorteil, dass die Flussrate automatisch an die aktuelle Behandlung angepasst beziehungsweise eingestellt werden kann, die Behandlung entsprechend nicht unnötig verzögert wird und nach einer vorgegebenen Verschreibung oder einem Behandlungsplan folgend erfolgt. Es kann jedoch auch vorgesehen sein, dass die Benutzereingabe eine beabsichtigte Flussrate, die als Verabreichungsrate im Behandlungsplan vorgesehen ist, umfasst, so dass diese entweder gleichzeitig als Bestätigung der Benutzeraufforderung dient oder eine redundante Sicherheit darstellt. Die eingegebene Flussrate beziehungsweise beabsichtigte Flussrate kann ebenfalls mit einem vorgegebenen Flusszustand oder einem Behandlungsparameter verglichen werden, sodass die Steuereinheit das Verschlusselement beim erheblichen Abweichen der Eingabe von beispielsweise dem vorgegebenen Flusszustand entsprechend betätigt und geschlossen hält und ein weiteres Signal ausgibt. Wenn die Benutzereingabe jedoch im Wesentlichen mit dem vorgegebenen Flusszustand übereinstimmt, so kann die Steuereinheit das Verschlusselement öffnen und die eingegebene Flussrate entsprechend einstellen.

Das Ändern beziehungsweise Einstellen der Flussrate kann durch die Betätigung des Verschlusselements oder auch einer entsprechenden Pumpe erfolgen. Entsprechend kann auch vorgesehen sein, dass die Flussrate direkt über die Pumpe eingestellt wird, wobei eine Flussrate gleich Null ebenfalls bewirkt, dass der zugegebene Stoff nicht verabreicht wird. Somit kann das Verschlusselement alternativ über eine entsprechende Ausgestaltung der Pumpe und der Steuereinheit durch die Pumpe ersetzt werden.

Weiterhin kann die Steuereinheit dazu eingerichtet sein, ein Alarmsignal beim Vorliegen eines Flusssignals auszugeben. Somit wird der Benutzer beispielsweise auf das Vorliegen eines Fehlers aufmerksam gemacht, sodass dieser die extrakorporale Blutbehandlungsvorrichtung und insbesondere die eine oder mehrere Anschlussstellen überprüft. Bevorzugt ist das Alarmsignal ein optisches und/oder akustisches Alarmsignal. Beispielsweise kann eine Warnleuchte blinken und oder ein Summton ertönen, sobald der detektierte Stofffluss anzeigt, dass ein Fehler in der extrakorporalen Blutbehandlungsvorrichtung vorliegt und die Sicherheit des Patienten möglicherweise gefährdet wird. Bevorzugt wird das Alarmsignal basierend auf einem Vergleich einer Benutzereingabe mit einem für die Anschlussstelle vorgegebenen Flusszustand ausgegeben. Entsprechend kann der Benutzer unmittelbar auf eine Abweichung vom vorgegebenen Flusszustand hingewiesen werden, sodass beispielsweise der Fehler beseitigt oder der vorgegebene Flusszustand angepasst werden kann.

Um das Detektieren des Stoffflusses und eine Ankopplung an eine jeweilige Anschlussstelle weiter zu vereinfachen, kann weiterhin vorgesehen sein, dass das Detektieren des Stoffflusses das Erfassen eines Koppelelements an der Anschlussstelle umfasst, wobei das Erfassen bevorzugt das Auslesen eines an dem Koppelelement angebrachten Kennzeichens und/oder eine mechanische, eine elektromechanische oder elektromagnetische Schaltung umfasst.

Beispielsweise kann die Detektionsanordnung ein jeweiliges Auslesegerät in Form eines Scanners oder Barcodescanners aufweisen, welcher im angekoppelten Zustand einer Zugabevorrichtung, beispielsweise einer Spritze oder eines Reservoirs oder Behälters, an einer Anschlussstelle ein benachbartes Kennzeichen ausliest. Das Kennzeichen kann auch in Form eines RFID ausgelesen werden. Je nach Ausgestaltung der extrakorporalen Blutbehandlungsvorrichtung kann diese Funktion auch von der Steuervorrichtung übernommen werden.

Alternativ kann vorgesehen sein, dass durch die Kopplung beispielsweise eine mechanische, elektromechanische oder elektromagnetische Schaltung betätigt wird. Beispielsweise kann das Koppelelement ein elektrisch leitfähiges Element aufweisen, was im eingesetzten Zustand an einer Anschlussstelle eine Schaltung bewirkt, sodass ein entsprechendes elektrisches Signal ausgelesen werden kann. Alternativ kann beispielsweise ein Hall-Element vorgesehen sein, sodass ein entsprechendes Signal durch Induktion bewirkt wird, beispielsweise wenn das Hall-Element im eingesetzten Zustand an einer Anschlussstelle ein elektromagnetisches Signal in einer sich in der Anschlussstelle befindenden Spule induziert.

Für bestimmte Anschlüsse können ebenfalls nur übereinstimmende Koppelelemente vorgesehen sein, sodass für diese Anschlüsse nur eine begrenzte Anzahl von zugegebenen Stoffen oder sogar nur ein Stoff vorgesehen ist. Zum Beispiel können das Koppelelement und die Anschlussstelle derart aneinander angepasst sein, dass diese in gekoppeltem Zustand eine formschlüssige Verbindung bilden, wobei dies für anders geformte Koppelelemente nicht zutrifft. Beispielsweise können das Koppelelement und die Anschlussstelle als entsprechende Poka Yoke Elemente ausgebildet sein. Entsprechend kann vorgesehen sein, dass eine Erfassung des Koppelelements bei übereinstimmenden Elementen erfolgt.

Bevorzugt handelt es sich bei der extrakorporalen Blutbehandlungsvorrichtung um eine Hämodialysevorrichtung, Hämofiltrationsvorrichtung oder Hämodiafiltrationsvorrichtung oder eine Vorrichtung zur therapeutischen Apherese. Entsprechend weist die extrakorporale Blutbehandlungsvorrichtung zumindest einen Dialysefilter auf, welcher einen extrakorporalen Blutkreislauf des Patienten von einem Dialysatkreislauf trennt und wobei zu entfernende Moleküle im Blut des Patienten über eine semipermeable Membran des Dialysefilters in das Dialysat diffundieren können, um somit eine Blutreinigung des Patienten zu bewirken. Bei einer Vorrichtung zur therapeutischen Apherese weist die extrakorporale Blutbehandlungsvorrichtung mindestens eine Adsorptionseinheit auf, die mit einem Dialysator kombiniert sein kann.

Für das Beispiel der Hämodialysevorrichtung ist bevorzugt die mindestens eine Anschlussstelle an einer Blutsseite und/oder einer Dialysatseite des Fluidsystems der extrakorporalen Blutbehandlungsvorrichtung angeordnet. Beispielsweise können beim Vorliegen von mehreren Anschlussstellen eine oder mehrere Anschlussstellen an der Blutseite des Fluidsystems angeordnet sein, während eine oder mehrere Anschlussstellen ebenfalls an der Dialysatseite angeordnet sein können. Die Überwachung der jeweiligen Anschlussstellen über die jeweiligen Flusszustände ist dabei besonders vorteilhaft, da bei einer versehentlichen Verwendung einer Anschlussstelle automatisch ein Signal ausgegeben und der Benutzer somit unmittelbar auf eine falsche Konfiguration aufmerksam gemacht wird. Somit kann beispielsweise verhindert werden, dass Stoffe, welche für eine Blutseite vorgesehen sind, nicht in die Dialysatseite infundiert werden und umgekehrt kein Stoff, der für die Dialysatseite vorgesehen ist, in den Blutkreis des Patienten gelangt. Ebenfalls kann somit sichergestellt werden, dass der Stoff an eine vorgesehene Pumpe gekoppelt wird, sodass eine vorgesehene Flussrate oder Flussrate ebenfalls eingehalten wird.

Bevorzugt umfasst das Fluidsystem der extrakorporalen Blutbehandlungsvorrichtung einen Einmalartikel und die Anschlussstelle ist an dem Einmalartikel angeordnet, wobei der Einmalartikel bevorzugt einen Behälter, ein Reservoir, einen Schlauch, einen Filter, einen Dialysefilter, einen Adsorber, eine Kassette und/oder einen Katheter umfasst. Beispielsweise kann ein Einmalartikel für eine Hämodialysevorrichtung in Form eines Schlauchsets ausgebildet sein, wobei das Schlauchset eine oder mehrere Anschlussstellen aufweist. Entsprechend können die Detektionsanordnung und die Steuervorrichtung auch in einem Einmalartikel integriert sein, sodass generell eine Überwachung von Anschlussstellen auch getrennt von einer extrakorporalen Blutbehandlungsvorrichtung und für andere Zwecke, beispielsweise Infusionen, erfolgen kann.

Die oben gestellte Aufgabe wird weiterhin durch ein Verfahren zum Überwachen einer Anschlussstelle mit den Merkmalen des Anspruchs 11 gelöst. Vorteilhafte Weiterbildungen des Verfahrens ergeben sich aus den Unteransprüchen sowie der vorliegenden Beschreibung und den Figuren.

Entsprechend wird ein Verfahren zum Überwachen einer Anschlussstelle einer extrakorporalen Blutbehandlungsvorrichtung, bevorzugt einer Hämodialysevorrichtung, Hämofiltrationsvorrichtung oder Hämodiafiltrationsvorrichtung oder Vorrichtung zur therapeutischen Apherese vorgeschlagen, wobei die extrakorporale Blutbehandlungsvorrichtung mindestens eine Anschlussstelle zum Zugeben von Stoffen aufweist, aufweisend die Schritte:
- Detektieren des Vorliegens eines Stoffflusses an der Anschlussstelle; und
- Ausgeben eines Signals bei Vorliegen eines Stoffflusses.

Das Verfahren wird dabei an einem vorstehend beschriebenen Fluidsystem vorgenommen, um entsprechende Anschlussstellen des Fluidsystems zu überwachen. Dabei kann der Stofffluss beispielsweise von einer Detektionsanordnung detektiert und der detektierte Stofffluss von einer Steuereinheit empfangen und verarbeitet beziehungsweise ausgelesen werden, wie vorstehend beschrieben. Insbesondere können somit sämtliche Verfahrensschritte durch die erfindungsgemäße Blutbehandlungsvorrichtung ausgeführt werden, sodass sowohl auf die entsprechenden, strukturellen Merkmale Bezug genommen und gleichzeitig auf eine detaillierte Beschreibung der entsprechenden Vorteile aus Redundanzgründen zumindest teilweise verzichtet wird.

Wenn die extrakorporale Blutbehandlungsvorrichtung mindestens zwei Anschlussstellen umfasst, werden gemäß dem Verfahren bevorzugt mindestens zwei Anschlussstellen überwacht, wobei das Vorliegen eines Stoffflusses einer der Anschlussstellen zugeordnet wird und wobei das ausgegebene Signal der zugeordneten Anschlussstelle entspricht.

Dadurch, dass, wenn mehrere Anschlussstellen vorgesehen sind, das Vorliegen eines Stoffflusses einer spezifischen Anschlussstelle zugeordnet werden kann, ist eine Überwachung der Anschlussstellen beziehungsweise der Zugabe von Stoffen auch in einem komplexeren System möglich. Somit wird dem Benutzer durch das Ausgeben eines Signals eine Rückkopplung beziehungsweise ein Feedback gegeben, welche(s) die verwendete Anschlussstelle bestätigt und/oder eine zu verwendende Anschlussstelle angibt. Der Benutzer wird somit an einem angehenden Fehler gehindert, sodass die Sicherheit des Patienten weiter erhöht wird, ohne dass es dabei kognitive Anstrengungen des Benutzers erfordert.

Bevorzugt wird das Signal basierend auf einem Vergleich des detektierten Stoffflusses mit einem für die jeweilige Anschlussstelle vorgegebenen Flusszustand ausgegeben.

Somit kann ein Signal ausgegeben werden, wenn aus dem Vergleich folgt, dass beispielsweise eine bestimmte Anschlussstelle nicht verwendet werden sollte, da ein entsprechender Flusszustand während des Betriebs der extrakorporalen Blutbehandlungsvorrichtung nicht vorgesehen ist.

Insofern kann ein solcher Vergleich bereits in einer einfachen Ausführung beziehungsweise bei nur einer Anschlussstelle den Vorteil haben, dass die Sicherheit des Patienten weiter verbessert wird. Bevorzugt werden jedoch mehrere Anschlussstellen überwacht, sodass ein Vergleich den weiteren Vorteil hat, dass nicht nur auf die jeweilige Verwendung der Anschlussstellen abgestellt, sondern optional ebenfalls geprüft werden kann, ob eine vorzunehmende Zugabe eines Stoffs mit dem entsprechenden vorgegebenen Flusszustand übereinstimmt. Mit anderen Worten kann für eine bestimmte Anschlussstelle beispielsweise die Zugabe eines bestimmten Medikaments vorgesehen sein, sodass das ausgegebene Signal eine Information bezüglich des entsprechenden Medikaments enthält.

Bevorzugt erfolgt das Detektieren des Stoffflusses durch das Erfassen eines Drucks und/oder eines Flusses mittels eines jeweiligen Drucksensors und/oder Flusssensors. Entsprechend kann beispielsweise durch die Erfassung eines absoluten Drucks oder Flusses bereits auf einen Stofffluss geschlossen werden, wobei die Erfassung bevorzugt über einen vorgegebenen Zeitraum erfolgt, um eventuelle Variationen einzuschließen und entsprechend beim Detektieren des Stoffflusses zu berücksichtigen.

Weiterhin umfasst das Signal bevorzugt das Anzeigen eines Hinweises mit einer Benutzeraufforderung auf einem Display, wobei ein an der Anschlussstelle vorgesehenes Verschlusselement basierend auf einer Benutzereingabe betätigt oder zumindest teilweise geöffnet und/oder wobei basierend auf einer Benutzereingabe ein weiteres Signal ausgegeben wird.

Entsprechend kann das Verfahren vorsehen, dass beispielsweise ein Verschlusselement zunächst geschlossen bleibt und erst nach der Eingabe eines Benutzers beziehungsweise nach dessen Bestätigung freigegeben wird. Der Benutzer bekommt somit nicht nur Feedback bezüglich der vorgenommenen Handlung, sondern muss diese ebenfalls bestätigen, bevorzugt mittels einer dem Stoff entsprechenden Eingabe.

Bevorzugt wird das Verschlusselement in Reaktion auf das Vorliegen eines Stoffflusses und/oder basierend auf einem Vergleich des detektierten Stoffflusses mit einem für die Anschlussstelle vorgegebenen Flusszustand betätigt, zumindest teilweise geöffnet und/oder es wird eine Flussrate entsprechend eingestellt. Beispielsweise kann die Eingabe die gewünschte Flussrate beziehungsweise beabsichtigte Flussrate umfassen. Sollte die Eingabe jedoch nicht mit einem vorgegebenen Flusszustand übereinstimmen, so kann das Verschlusselement weiterhin im geschlossenen Zustand gehalten werden, wobei zusätzlich ein weiteres Signal ausgegeben werden kann, um den Benutzer auf die fehlende Übereinstimmung aufmerksam zu machen.

Ebenfalls kann vorgesehen sein, dass ein Alarmsignal, bevorzugt ein optisches und/oder akustisches Alarmsignal, basierend auf dem detektierten Stofffluss, bevorzugt basierend auf einem Vergleich der Benutzereingabe mit einem für die Anschlussstelle vorgegebenen Flusszustand, ausgegeben wird. Entsprechend wird der Benutzer beispielsweise auf einen akuten Fehler hingewiesen, sodass dieser, anders als bei einem einfachen Feedback, die Anschlussstellen des Fluidsystems überprüfen muss.

Weiterhin kann das Detektieren des Stoffflusses das Erfassen eines Koppelelements an der Anschlussstelle umfassen, wobei das Erfassen bevorzugt das Auslesen eines an dem Koppelelement angebrachten Kennzeichens und/oder eine mechanische, eine elektromechanische oder elektromagnetische Schaltung umfasst. Entsprechend wird der Stofffluss bereits beim Ankoppeln automatisch erfasst und es kann beispielsweise auf eine komplexere Verarbeitung eines detektierten Stoffflusses durch beispielsweise eine Detektionsanordnung verzichtet werden.

### Kurze Beschreibung der Figuren

Bevorzugte weitere Ausführungsformen der Erfindung werden durch die nachfolgende Beschreibung der Figuren näher erläutert. Dabei zeigen:
- Figur 1: eine extrakorporale Blutbehandlungsvorrichtung mit einer Anschlussstelle;
- Figur 2: eine extrakorporale Blutbehandlungsvorrichtung mit mehreren Anschlussstellen;
- Figur 3: eine extrakorporale Blutbehandlungsvorrichtung gemäß Figur 2 mit einem Display;
- Figur 4: eine extrakorporale Blutbehandlungsvorrichtung gemäß Figur 3 mit Verschlusselementen und einer weiteren Benutzerrückkopplung;
- Figur 5: eine extrakorporale Blutbehandlungsvorrichtung gemäß Figur 1 mit einem angeschlossenen Koppelelement; und
- Figur 6: ein Hämodialysegerät mit einer Blutseite und einer Dialysatseite und mit angekoppelten Einmalartikeln.

### Detaillierte Beschreibung bevorzugter Ausführungsbeispiele

Im Folgenden werden bevorzugte Ausführungsbeispiele anhand der Figuren beschrieben. Dabei werden gleiche, ähnliche oder gleichwirkende Elemente in den unterschiedlichen Figuren mit identischen Bezugszeichen versehen. Auf eine wiederholte Beschreibung dieser Elemente wird teilweise verzichtet, um Redundanzen zu vermeiden.

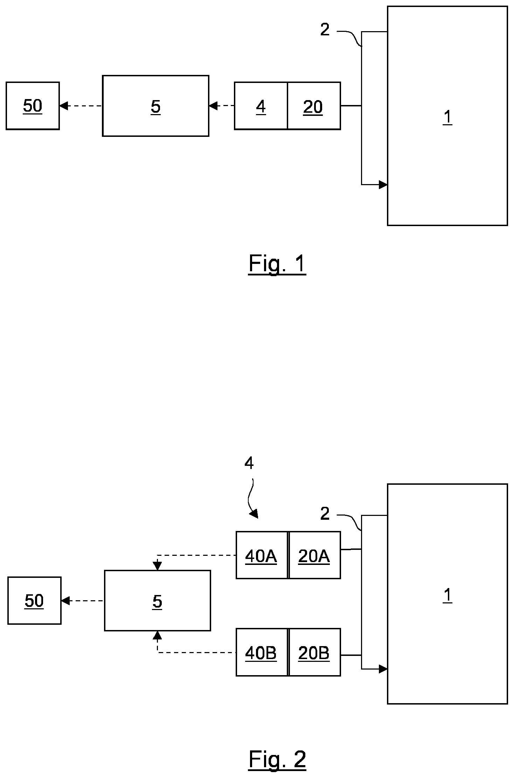

In Figur 1 ist schematisch eine extrakorporale Blutbehandlungsvorrichtung 1 gezeigt, welche ein Fluidsystem 2 aufweist. Das Fluidsystem 2 ist an einem Ausgang und an einem Eingang der extrakorporalen Blutbehandlungsvorrichtung 1 mittels einer Fluidkopplung gekoppelt und bildet somit einen Kreislauf, wie schematisch mit dem Pfeil dargestellt. Das Fluidsystem 2 kann auch den gesamten Kreislauf in der extrakorporalen Blutbehandlungsvorrichtung ausbilden und beispielsweise in Form eines Schlauchsets ausgebildet sein, welches in die extrakorporale Blutbehandlungsvorrichtung 1 eingesetzt ist.

An dem Fluidsystem 2 ist eine Anschlussstelle 20 angeordnet, welche das Zugeben von Stoffen in das Fluidsystem 2 ermöglicht. Beispielsweise können somit Medikamente oder generell Flüssigkeiten mit einer therapeutischen Wirkung für einen an der extrakorporalen Blutbehandlungsvorrichtung 1 angeschlossenen Patienten (nicht gezeigt), entweder mittelbar oder unmittelbar, zugegeben werden. Die Anschlussstelle 20 ist vorliegend als ein Reservoir mit einer Luer-Lock Verbindung ausgebildet, sodass beispielsweise eine Spritze mit einer entsprechenden Kopplung einfach an die Anschlussstelle 20 angekoppelt werden kann. Das Reservoir bildet dabei einen Hohlraum und kann entweder bereits eine Flüssigkeit enthalten oder zumindest teilweise mit Luft gefüllt sein, um somit ein vorzeitiges Eindringen der zugegebenen Flüssigkeiten zu verhindern beziehungsweise einen Gegendruck aufzubauen, welcher nach entsprechender Zugabe verdrängt werden kann. Anstatt eines Reservoirs mit einer spezifischen Verbindung kann jedoch generell jede Anschlussstelle 20 gewählt werden, beispielsweise auch ein einfacher Schlauch und/oder eine selbstschließende Membran.

Die Anschlussstelle 20 ist mit einer Detektionsanordnung 4 verbunden, sodass über die Detektionsanordnung 4 ein Stofffluss an der Anschlussstelle 20 detektiert werden kann. Entsprechend kann beispielsweise erfasst werden, ob die Anschlussstelle aktiv benutzt beziehungsweise verwendet wird oder sich in einem freien beziehungsweise entkoppelten Zustand befindet. Diese Information wird über ein Flusssignal an eine Steuereinheit 5 weitergeleitet, wobei die Steuereinheit 5 dazu eingerichtet ist, in Reaktion auf den Empfang eines Flusssignals ein entsprechendes Signal 50 auszugeben, wie mit den gestrichelten Pfeilen dargestellt.

Gemäß der vorliegenden Ausführungsform ist das Signal ein optisches Signal in Form einer Leuchte, beispielsweise eine LED, welche an der Anschlussstelle 20 angeordnet ist. Folglich kann dem Benutzer über das Signal 50 unmittelbar beziehungsweise nach Verarbeitung des detektierten Stoffflusses ein Feedback bezüglich der vorgenommenen Handlung gegeben werden, sodass dieser eine vorgenommene Zugabe eines Stoffs beziehungsweise eine Ankopplung an die Anschlussstelle 20 entweder erneut überprüft oder dass sichergestellt wird, dass die Ankopplung von der Steuereinheit 5 erfasst wurde. Somit wird eine einfache Überwachung der vorgenommenen Handlungen während und/oder vor der Behandlung des Patienten bereitgestellt.

In der Ausführungsform gemäß Figur 2 sind im Fluidsystem 2 der extrakorporalen Blutbehandlungsvorrichtung 1 mehrere Anschlussstellen 20A, 20B vorgesehen. An jeder Anschlussstelle 20A, 20B ist eine jeweilige Detektionseinheit der Detektionsanordnung 4 vorgesehen, welche vorliegend als Drucksensor 40A beziehungsweise Durchflusssensor 40B ausgebildet sind. Entsprechend wird ein Stofffluss der Anschlussstelle 20A über eine Messung des Drucks bestimmt, wobei die Erfassung des Drucks über einen vorgegebenen Zeitraum erfolgt und wobei die Werte entsprechend gemittelt werden beziehungsweise eine Variation der Werte ausgewertet wird. Entsprechend wird auch ein Stofffluss der Anschlussstelle 20B bestimmt, wobei es sich hierbei nicht um den Druck, sondern um den gemessenen Durchfluss handelt.

Die Flusssignale werden von der Steuereinheit 5 empfangen und einer bestimmten beziehungsweise spezifischen Anschlussstelle 20A, 20B zugeordnet, sodass die Steuereinheit 5 zwischen dem Flusszustand der Anschlussstelle 20A und dem Flusszustand der Anschlussstelle 20B unterscheiden kann. Weiterhin sind in der Steuereinheit 5 vorgegebene Werte der Flusszustände für die jeweilige Anschlussstelle 20A, 20B hinterlegt, sodass die Steuereinheit 5 den über das Detektionssignal empfangenen Flusszustand entsprechend mit einem Soll-Wert oder einem vorgegebenen Bereich vergleichen kann.

Wenn der detektierte Stofffluss in dem vorgegebenen Bereich liegt oder einem Soll-Wert entspricht, so kann in Reaktion darauf ein entsprechendes Signal 50 ausgegeben werden, wodurch der Benutzer eine implizite Bestätigung der korrekten Ankopplung an der jeweiligen Anschlussstelle 20A, 20B erhält. Wenn der Flusszustand jedoch von dem vorgegebenen Flusszustand abweicht, so kann entsprechend ein Signal ausgegeben werden, welches den Benutzer auf die fehlerhafte Ankopplung aufmerksam macht und gegebenenfalls eine Information über die korrekte beziehungsweise vorgesehene Ankopplung enthält.

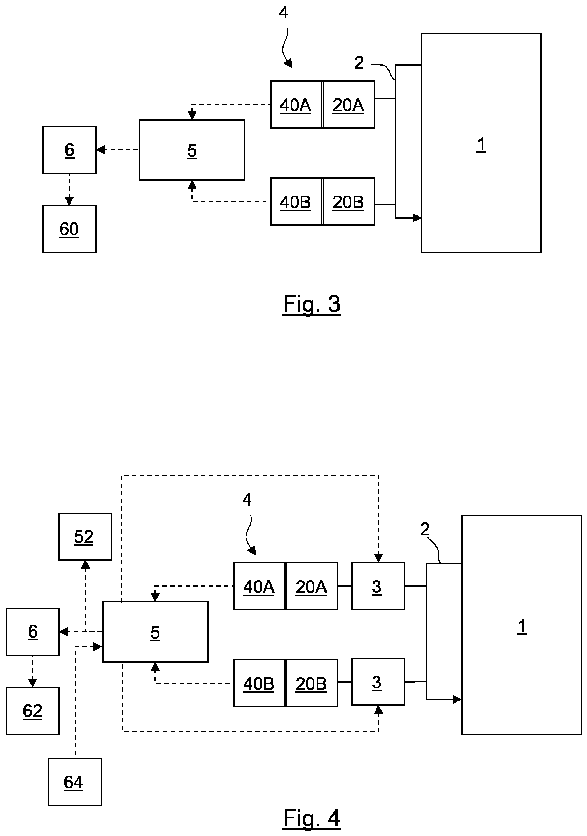

Die Ausführungsform gemäß Figur 3 entspricht im Wesentlichen der in Figur 2 dargestellten extrakorporalen Blutbehandlungsvorrichtung. Zusätzlich umfasst die extrakorporale Blutbehandlungsvorrichtung 1 ein Display 6, welches mit der Steuereinheit kommunikativ verbunden ist. Durch das Display 6 ist das Ausgeben eines Signals in der Form eines detaillierteren Hinweises 60 möglich, welcher zusätzliche Informationen bezüglich des Stoffflusses der jeweiligen Anschlussstellen 20A, 20B enthält. Beispielsweise kann der Hinweis 60 eine grafische Darstellung des aktuellen Stoffflusses einer jeweiligen Anschlussstelle 20A, 20B darstellen, zum Beispiel als Übersicht der jeweiligen Anschlussstellen 20A, 20B, wobei die entsprechende Position im System beziehungsweise in der extrakorporalen Blutbehandlungsvorrichtung 1 schematisch dargestellt und mit dem jeweiligen Stofffluss versehen ist. Somit kann der Hinweis 60 ebenfalls auf eine erwünschte oder erforderliche Änderung eine Ankopplung hinweisen, beispielsweise durch eine entsprechende Markierung der betroffenen Anschlussstellen 20A, 20B.

Weiterhin kann das Display 6 in dem grafisch dargestellten Hinweis 60 optional das Ergebnis eines Vergleichs des detektierten Stoffflusses mit einem für die jeweilige Anschlussstelle 20A, 20B vorgegebenen Flusszustand darstellen, wobei übereinstimmende und fehlerhafte Werte eine unterschiedliche Farbkodierung aufweisen können. Ebenfalls können die Werte der bestimmten und/oder vorgegebenen Flusszustände in dem Hinweis 60 dargestellt sein, sodass dem Benutzer angezeigt wird, inwiefern der detektierte Stofffluss mit dem vorgegebenen Stofffluss übereinstimmt.

In Figur 4 ist die extrakorporale Blutbehandlungsvorrichtung 1 gemäß Figur 3 weiterhin mit Verschlusselementen 3 einer weiteren Benutzerrückkopplung versehen. Jede der Anschlussstellen 20A, 20B weist dabei ein Verschlusselement 3 auf, welches eine Fluidkopplung der jeweiligen Anschlussstelle 20A, 20B mit einem zentralen Schlauch des Fluidsystems 2 bereitstellt. Die Verschlusselemente 3 gemäß der vorliegenden Ausführungsform sind jeweils als Klemme ausgebildet und verhindern im geschlossenen Zustand, dass ein an der jeweiligen Anschlussstelle 20A, 20B zugegebener Stoff in das Fluidsystem 2 einfließen kann. Die Verschlusselemente 3 sind dabei zunächst geschlossen und können über die Steuereinheit 5 betätigt werden.

Wird eine Anschlussstelle 20A, 20B betätigt beziehungsweise verwendet, um einen Stoff zuzugeben, so ändert sich der Stofffluss an der jeweiligen Anschlussstelle 20A, 20B, welcher von der Detektionsanordnung 4 bestimmt beziehungsweise erfasst wird. Dadurch, dass die Steuereinheit 5 den geänderten Stofffluss einer bestimmten Anschlussstelle 20A, 20B zuordnet, enthält der Hinweis auf dem Display 6 eine entsprechende Benutzeraufforderung 62, welche den Benutzer dazu auffordert, zu bestätigen, dass die verwendete Anschlussstelle 20A, 20B tatsächlich verwendet werden soll und kann dabei ebenfalls einen vorgegebenen Flusszustand anzeigen, beispielsweise einen für die jeweilige Anschlussstelle 20A, 20B vorgesehenen Stoff.

Der Benutzer gibt danach eine Benutzereingabe 64 ein, welche nur eine Bestätigung oder auch eine konkrete Eingabe des verwendeten Stoffs oder sogar eine erwünschte Flussrate für die Verabreichung des Stoffs aufweisen kann. Die Benutzereingabe 64 kann, wie dargestellt, direkt an der Steuereinheit 5 vorgenommen werden. Entsprechend wird die Benutzereingabe 64 in der Steuereinheit 5 hinterlegt und wird das betreffende Verschlusselement 3 entsprechend betätigt, sodass beispielsweise eine Klemme geöffnet und/oder eine Flussrate automatisch eingestellt wird.

Sollte die Benutzereingabe 64 jedoch nicht mit einem vorgegebenen Flusszustand oder Wert übereinstimmen oder von einem vorgegebenen Bereich abweichen, so kann die Steuereinheit 5 ein weiteres Signal 52 ausgeben, beispielsweise ein akustisches Alarm oder das Aufleuchten einer Leuchte, um den Benutzer auf die Abweichung aufmerksam zu machen und zu signalisieren, dass das jeweilige Verschlusselement 3 (noch) nicht betätigt wird.

Der Benutzer erhält somit eine erweiterte Rückkopplung beziehungsweise ein Feedback, welches verhindert, dass Stoffe versehentlich über die falsche Anschlussstelle 20A, 20B verabreicht beziehungsweise infundiert werden. Somit wird die Sicherheit des Patienten signifikant erhöht.

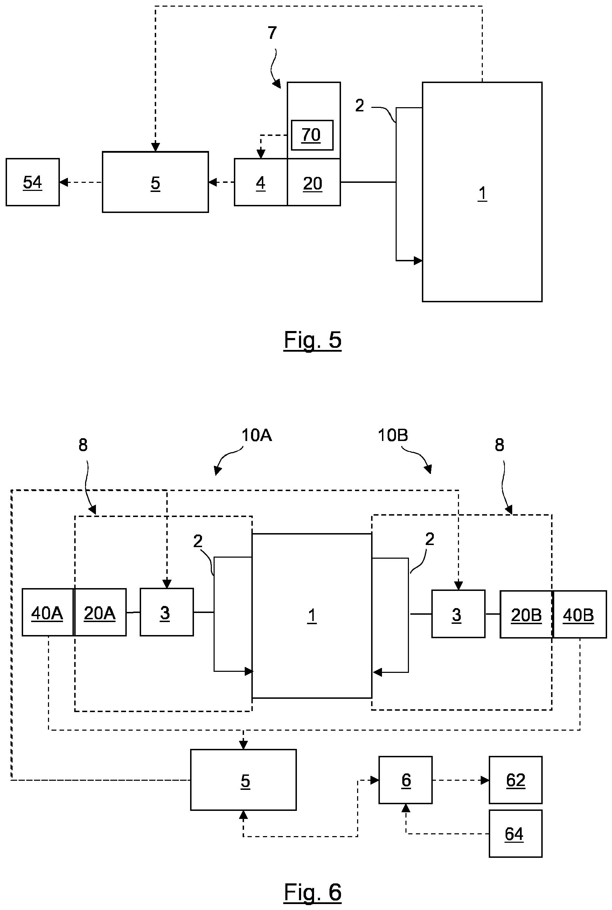

In der extrakorporalen Blutbehandlungsvorrichtung 1 gemäß Figur 5 ist die Ausführungsform gemäß Figur 1 mit einem angeschlossenen Koppelelement 7 versehen. Die Detektionsanordnung 4 ist dabei eingerichtet, ein an dem Koppelelement 7 angebrachtes Kennzeichen 70 auszulesen, wobei das Kennzeichen 70 in Form eines RFID ausgebildet ist. Entsprechend kann die Detektionsanordnung 4 dazu eingerichtet sein, die relevanten Informationen bezüglich des Koppelelements 7 und des entsprechenden Stoffs automatisch auszulesen beziehungsweise zu empfangen und diese entsprechend als Flusssignal an die Steuereinheit 5 weiterzuleiten.

Die Steuereinheit 5 ist dabei kommunikativ mit der extrakorporalen Blutbehandlungsvorrichtung 1 verbunden, sodass die Steuereinheit 5 den vorgegebenen Flusszustand anhand von mindestens einem erfassten Parameter in der extrakorporalen Blutbehandlungsvorrichtung 1 bestimmen und diesen mit dem detektierten Stofffluss vergleichen kann. Wenn der detektierte Stofffluss nicht mit dem vorgegebenen Flusszustand übereinstimmt, so kann die Steuereinheit 5 ein entsprechendes Alarmsignal 54 ausgeben, sodass der Benutzer unmittelbar auf die Ankopplung eines falschen Koppelelements 7 hingewiesen wird und die Anschlussstelle 20 entsprechend überprüfen kann, um den Fehler zu beseitigen beziehungsweise einen Behälter mit dem vorgesehenen Stoff anzukoppeln.

Die Ausführungsform gemäß Figur 6 entspricht im Wesentlichen der extrakorporalen Blutbehandlungsvorrichtung 1 gemäß Figur 4, wobei die extrakorporale Blutbehandlungsvorrichtung 1 als Hämodialysegerät mit einer Blutseite 10A und einer Dialysatseite 10B ausgebildet ist und wobei die Komponenten zumindest teilweise als angekoppelte Einmalartikel 8 ausgebildet sind.

Entsprechend werden an beiden Seiten 10A, 10B des Hämodialysegeräts die jeweiligen Fluidsysteme 2, die Verschlusselemente 3 und die Anschlussstellen 20A, 20B als Einmalartikel 8 ausgebildet. Der venöse Zugang und der arterielle Zugang an der Blutseite 10A sind dabei nicht gezeigt.

An die jeweiligen Anschlussstellen 20A, 20B der Einmalartikel 8 sind entsprechend die Sensoren 40A, 40B gekoppelt, sodass die Steuereinheit 5 Flusssignale bezüglich des detektierten Stoffflusses empfangen und verarbeiten beziehungsweise auswerten kann. Weiterhin ist die Steuereinheit 5 mit den jeweiligen Verschlusselementen 3 kommunikativ verbunden, sodass die Steuereinheit 5, wie in Figur 4, in Abhängigkeit von einer Benutzereingabe 64 die jeweiligen Verschlusselemente 3 betätigen kann. Das Eingeben der Benutzereingabe 64 erfolgt gemäß dieser Ausführungsform auf dem Display 6, welches das Eingeben der Benutzereingabe 64 ermöglicht, beispielsweise wenn dieses entsprechend als Touchpad ausgebildet ist oder eine Tastatur aufweist. Die Schnittstelle zwischen dem Display 6 und der Steuereinheit 5 ermöglicht in diesem Fall eine Kommunikation in beide Richtungen, sodass die Benutzereingabe 64 ebenfalls an die Steuereinheit 5 weitergeleitet wird.

Folglich kann die Steuereinheit 5 die Anschlussstellen 20A, 20B der jeweiligen Seite 10A, 10B überwachen und dabei zwischen den Seiten 10A, 10B unterscheiden, sodass durch die spezifische Zuordnung der Anschlussstellen 20A, 20B und die erforderliche Benutzereingabe 64 eine erhöhte Sicherheit des Patienten bereitgestellt und gleichzeitig eine korrekte Funktionsfähigkeit gemäß therapeutischen Vorschriften eingehalten werden.

Soweit anwendbar, können alle einzelnen Merkmale, die in den Ausführungsbeispielen dargestellt sind, miteinander kombiniert und/oder ausgetauscht werden, ohne den Bereich der Erfindung zu verlassen.

### Bezugszeichenliste

- 1: Extrakorporale Blutbehandlungsvorrichtung
- 10A: Blutseite
- 10B: Dialysatseite
- 2: Fluidsystem
- 20: Anschlussstelle
- 20A: Anschlussstelle
- 20B: Anschlussstelle
- 3: Verschlusselement
- 4: Detektionsanordnung
- 40A: Drucksensor
- 40B: Durchflusssensor
- 5: Steuereinheit
- 50: Signal
- 52: Signal
- 54: Alarmsignal
- 6: Display
- 60: Hinweis
- 62: Benutzeraufforderung
- 64: Benutzereingabe
- 7: Koppelelement
- 70: Kennzeichen
- 8: Einmalartikel

## Patentansprüche

1. Extrakorporale Blutbehandlungsvorrichtung (1), bevorzugt Hämodialysevorrichtung, Hämofiltrationsvorrichtung oder Hämodiafiltrationsvorrichtung, umfassend:
- ein Fluidsystem (2) mit einer Anschlussstelle (20) zum Zugeben von Stoffen;
- eine Detektionsanordnung (4) zum Detektieren eines Stoffflusses an der Anschlussstelle (20), wobei die Detektionsanordnung dazu eingerichtet ist, ein Flusssignal zu erzeugen, wenn ein Stofffluss an der Anschlussstelle (20) detektiert wird;
- eine Steuereinheit (5), welche mit der Detektionsanordnung (4) kommunizierend verbunden ist und dazu eingerichtet ist, Flusssignale zu empfangen und beim Vorliegen eines Flusssignals ein Signal (50), welches auf eine Verwendung der Anschlussstelle (20) hinweist, auszugeben.

2. Extrakorporale Blutbehandlungsvorrichtung (1) gemäß Anspruch 1, **dadurch gekennzeichnet, dass** das Fluidsystem (2) mindesten zwei Anschlussstellen (20A, 20B) zum Zugeben von Stoffen aufweist, wobei die Steuereinheit (5) dazu eingerichtet ist, ein Flusssignal einer der Anschlussstellen (20A, 20B) zuzuordnen und das Signal (50) entsprechend der zugeordneten Anschlussstelle (20A, 20B) auszugeben.

3. Extrakorporale Blutbehandlungsvorrichtung (1) gemäß Anspruch 1 oder 2, **dadurch gekennzeichnet, dass** die Detektionsanordnung (4) für jede Anschlussstelle (20A, 20B) jeweils einen Drucksensor (40A) und/oder einen Durchflusssensor (40B) umfasst und das Detektieren des Stoffflusses das Erfassen eines Drucks und/oder eines Flusses umfasst.

4. Extrakorporale Blutbehandlungsvorrichtung (1) gemäß einem der vorherigen Ansprüche, **dadurch gekennzeichnet, dass** ein Display (6) vorgesehen ist und das von der Steuereinheit (5) ausgegebene Signal (50) die Ausgabe eines Hinweises (60) auf dem Display (6) umfasst.

5. Extrakorporale Blutbehandlungsvorrichtung (1) gemäß einem der vorstehenden Ansprüche, **dadurch gekennzeichnet, dass** mindestens eine der Anschlussstellen (20A, 20B) ein Verschlusselement (3), bevorzugt eine Klemme, aufweist, wobei jedes der Verschlusselemente (3) mit der Steuereinheit (5) kommunikativ verbunden ist und wobei die Steuereinheit (5) dazu eingerichtet ist, eine Benutzeraufforderung (62) in Reaktion auf das Vorliegen des Signals (50) auszugeben und die Steuereinheit (5) dazu eingerichtet ist, das Verschlusselement (3) basierend auf einer Benutzereingabe (64) zu betätigen oder zumindest teilweise zu öffnen und/oder basierend auf einer Benutzereingabe (64) ein weiteres Signal (52) auszugeben.

6. Extrakorporale Blutbehandlungsvorrichtung (1) gemäß Anspruch 5, **dadurch gekennzeichnet, dass** die Steuereinheit (5) dazu eingerichtet ist, das Verschlusselement (3) bei Vorliegen des Flusssignals nach einem Vergleich mit einem für die Anschlussstelle (20A, 20B) vorgegebenen Flusszustand zu betätigen oder zumindest teilweise zu öffnen.

7. Extrakorporale Blutbehandlungsvorrichtung (1) gemäß Anspruch 5 oder 6, **dadurch gekennzeichnet, dass** die Steuereinheit (5) dazu eingerichtet ist, bei Vorliegen des Flusssignals für die Anschlussstelle (20A, 20B) eine Flussrate, basierend auf einem Vergleich einer vorliegenden Flussrate mit einer für die Anschlussstelle (20A, 20B) vorgegebenen Flussrate und/oder basierend auf einer Benutzereingabe (64), einzustellen.

8. Extrakorporale Blutbehandlungsvorrichtung (1) gemäß einem der vorherigen Ansprüche, **dadurch gekennzeichnet, dass** die Steuereinheit (5) dazu eingerichtet ist, ein Alarmsignal (54), bevorzugt ein optisches und/oder akustisches Alarmsignal, beim Vorliegen eines Flusssignals auszugeben, bevorzugt basierend auf einem Vergleich einer Benutzereingabe mit einem für die Anschlussstelle (20) vorgegebenen Flusszustand.

9. Extrakorporale Blutbehandlungsvorrichtung (1) gemäß einem der vorherigen Ansprüche, **dadurch gekennzeichnet, dass** das Detektieren eines Stoffflusses das Erfassen eines Koppelelements (7) an der Anschlussstelle (20) umfasst, wobei das Erfassen bevorzugt das Auslesen eines an dem Koppelelement (7) angebrachten Kennzeichens (70) und/oder eine mechanische, eine elektromechanische oder elektromagnetische Schaltung umfasst.

10. Extrakorporale Blutbehandlungsvorrichtung (1) gemäß einem der vorherigen Ansprüche, **dadurch gekennzeichnet, dass** das Fluidsystem (2) einen Einmalartikel (8) umfasst und die Anschlussstelle (20A, 20B) an dem Einmalartikel (8) angeordnet ist, wobei der eine Einmalartikel (8) bevorzugt einen Behälter, ein Reservoir, einen Schlauch, einen Filter, einen Dialysefilter, eine Adsorptionseinheit, eine Kassette und/oder einen Katheter umfasst.

11. Verfahren zum Überwachen einer Anschlussstelle (20A, 20B) einer extrakorporalen Blutbehandlungsvorrichtung (1), bevorzugt einer Hämodialysevorrichtung, Hämofiltrationsvorrichtung oder Hämodiafiltrationsvorrichtung, wobei die extrakorporale Blutbehandlungsvorrichtung (1) mindestens eine Anschlussstelle (20A, 20B) zum Zugeben von Stoffen in einem Fluidsystem (2) der extrakorporalen Blutbehandlungsvorrichtung (1) aufweist, aufweisend die Schritte:
- Detektieren des Vorliegens eines Stoffflusses an der Anschlussstelle (20A, 20B); und
- Ausgeben eines Signals (50) bei Vorliegen eines Stoffflusses, wobei das Signal (50) auf eine Verwendung der Anschlussstelle (20A, 20B) hinweist.

12. Verfahren gemäß Anspruch 11, **dadurch gekennzeichnet, dass** mindestens zwei Anschlussstellen (20A, 20B) vorgesehen sind und das Vorliegen eines Stoffflusses einer der Anschlussstellen zugeordnet wird, wobei das ausgegebene Signal (50) der zugeordneten Anschlussstelle (20A, 20B) entspricht.

13. Verfahren gemäß Anspruch 11 oder 12, **dadurch gekennzeichnet, dass** das Signal (50) basierend auf einem Vergleich des detektierten Stoffflusses mit einem für die jeweilige Anschlussstelle (20A, 20B) vorgegebenen Flusszustand ausgegeben wird.

14. Verfahren gemäß einem der Ansprüche 11 bis 13, **dadurch gekennzeichnet, dass** das Signal das Anzeigen eines Hinweises mit einer Benutzeraufforderung auf einem Display umfasst und wobei ein an der Anschlussstelle vorgesehenes Verschlusselement basierend auf einer Benutzereingabe betätigt oder zumindest teilweise geöffnet wird und/oder wobei basierend auf einer Benutzereingabe ein weiteres Signal ausgegeben wird.

15. Verfahren gemäß einem der Ansprüche 11 bis 14, **dadurch gekennzeichnet, dass** das Detektieren eines Stoffflusses das Erfassen eines Koppelelements an der Anschlussstelle (20A, 20B) umfasst, wobei das Erfassen bevorzugt das Auslesen eines an dem Koppelelement angebrachten Kennzeichens und/oder eine mechanische, eine elektromechanische oder elektromagnetische Schaltung umfasst.

## Claims

1. Extracorporeal blood treatment device (1), preferably a haemodialysis device, haemofiltration device or haemodiafiltration device, comprising:
- a fluid system (2) with a connection point (20) for the addition of substances;
- a detection arrangement (4) for detecting a substance flow at the connection point (20), wherein the detection arrangement is configured to generate a flow signal when a substance flow is detected at the connection point (20);
- a control unit (5), which is connected to communicate with the detection arrangement (4) and is configured to receive flow signals and, in the presence of a flow signal, to output a signal (50) which indicates that the connection point (20) is being used.

2. Extracorporeal blood treatment device (1) according to claim 1, **characterised in that** the fluid system (2) has at least two connection points (20A, 20B) for the addition of substances, wherein the control unit (5) is configured to associate a flow signal with one of the connection points (20A, 20B) and to output the signal (50) according to the associated connection point (20A, 20B).

3. Extracorporeal blood treatment device (1) according to claim 1 or 2, **characterised in that** the detection arrangement (4) respectively comprises a pressure sensor (40A) and/or a flow sensor (40B) for each connection point (20A, 20B) and wherein detecting the substance flow comprises determining a pressure and/or a flow.

4. Extracorporeal blood treatment device (1) according to any one of the preceding claims, **characterised in that** a display (6) is provided and wherein the signal (50) output by the control unit (5) comprises the output of an indication (60) on the display (6).

5. Extracorporeal blood treatment device (1) according to any one of the preceding claims, **characterised in that** at least one of the connection points (20A, 20B) comprises a closure element (3), preferably a clamp, wherein each of the closure elements (3) is communicatively connected to the control unit (5), and wherein the control unit (5) is configured to output a user prompt (62) in response to the presence of the signal (50) and wherein the control unit (5) is configured to actuate or at least partially open the closure element (3) based on a user input (64) or at least and/or to output a further signal (52) based on a user input (64).

6. Extracorporeal blood treatment device (1) according to claim 5, **characterised in that** the control unit (5) is configured to actuate or at least partially open the closure element (3) in the presence of the flow signal after a comparison with a predetermined flow state for the connection point (20A, 20B).

7. Extracorporeal blood treatment device (1) according to claim 5 or 6, **characterised in that** the control unit (5) is configured, in the presence of the flow signal, to set a flow rate for the connection point (20A, 20B) based on a comparison of a present flow rate with a predetermined flow rate for the connection point (20A, 20B) and/or based on a user input (64).

8. Extracorporeal blood treatment device (1) according to any one of the preceding claims, **characterised in that** the control unit (5) is configured to output an alarm signal (54), preferably an optical and/or acoustic alarm signal, when a flow signal is present, preferably based on a comparison of a user input with a predetermined flow state for the connection point (20).

9. Extracorporeal blood treatment device (1) according to any one of the preceding claims, **characterised in that** the detection of a substance flow comprises determining a coupling element (7) at the connection point (20), wherein the determining preferably comprises reading an identifier (70) attached to the coupling element (7) and/or a mechanical, electromechanical or electromagnetic circuit.

10. Extracorporeal blood treatment device (1) according to any one of the preceding claims, **characterised in that** the fluid system (2) comprises a disposable article (8) and the connection point (20A, 20B) is arranged on the disposable article (8), wherein the one disposable article (8) preferably comprises a container, a reservoir, a tube, a filter, a dialysis filter, an adsorption unit, a cassette and/or a catheter.

11. Method for monitoring a connection point (20A, 20B) of an extracorporal blood treatment device (1), preferably a haemodialysis device, haemofiltration device or haemodiafiltration device, wherein the extracorporal blood treatment device (1) has at least one connection point (20A, 20B) for the addition of substances into a fluid system (2) of the extracorporal blood treatment device (1), comprising the steps:
- detecting the presence of a substance flow at the connection point (20A, 20B); and
- outputting a signal (50) when a substance flow is present, wherein the signal (50) indicates a use of the connection point (20A, 20B).

12. Method according to claim 11, **characterised in that** at least two connection points (20A, 20B) are provided and the presence of a substance flow is associated with one of the connection points, wherein the output signal (50) corresponds to the associated connection point (20A, 20B).

13. Method according to claim 11 or 12, **characterised in that** the signal (50) is output based on a comparison of the detected substance flow with a predetermined flow state for the respective connection point (20A, 20B).

14. Method according to any one of claims 11 to 13, **characterised in that** the signal comprises displaying an indication with a user prompt on a display, and wherein a closure element provided at the connection point is actuated or at least partially opened based on a user input and/or wherein a further signal is output based on a user input.

15. Method according to any one of claims 11 to 14, **characterised in that** the detection of a substance flow comprises determining a coupling element at the connection point (20A, 20B), wherein the determining preferably comprises reading an identifier attached to the coupling element and/or a mechanical, electromechanical or electromagnetic circuit.

## Revendications

1. Dispositif de traitement extracorporel du sang (1), de préférence dispositif d'hémodialyse, dispositif d'hémofiltration ou dispositif d'hémodiafiltration comprenant :
- un système de fluide (2) avec un point de raccordement (20) pour l'ajout de substances ;
- un agencement de détection (4) pour la détection d'un flux de substance au niveau du point de raccordement (20), dans lequel l'agencement de détection est conçu afin de générer un signal de flux lorsqu'un flux de substance est détecté au niveau du point de raccordement (20) ;
- un dispositif de commande (5) qui est relié de manière communicante avec l'agencement de détection (4) et conçu afin de recevoir des signaux de flux et d'émettre un signal (50) qui indique une utilisation du point de raccordement (20) en présence d'un signal de flux.

2. Dispositif de traitement extracorporel du sang (1) selon la revendication 1, **caractérisé en ce que** le système de fluide (2) présente au moins deux points de raccordement (20A, 20B) pour l'ajout de substances, dans lequel le dispositif de commande (5) est conçu afin d'associer un signal de flux à l'un des points de raccordement (20A, 20B) et d'émettre le signal (50) selon le point de raccordement associé (20A, 20B).

3. Dispositif de traitement extracorporel du sang (1) selon la revendication 1 ou 2, **caractérisé en ce que** l'agencement de détection (4) pour chaque point de raccordement (20A, 20B) comprend respectivement un capteur de pression (40) et/ou un capteur de débit (40B) et la détection du flux de substance comprend la détection d'une pression et/ou d'un flux.

4. Dispositif de traitement extracorporel du sang 1) selon l'une quelconque des revendications précédentes, **caractérisé en ce qu'**un écran (6) est prévu et le signal (50) émis par le dispositif de commande (5) comprend l'émission d'une indication (60) sur l'écran (6).

5. Dispositif de traitement extracorporel du sang (1) selon l'une quelconque des revendications précédentes, **caractérisé en ce qu'**au moins un des points de raccordement (20A, 20B) présente un élément de fermeture (3), de préférence une pince, dans lequel chacun des éléments de fermeture (3) est relié de manière communicative au dispositif de commande (5) et dans lequel le dispositif de commande (5) est conçu afin d'émettre une invite utilisateur (62) en réaction à la présence du signal (50) et le dispositif de commande (5) est conçu afin d'actionner l'élément de fermeture (3) sur la base d'une entrée utilisateur (64) ou de l'ouvrir au moins partiellement et/ou d'émettre un autre signal (52) sur la base d'une entrée utilisateur (64).

6. Dispositif de traitement extracorporel du sang (1) selon la revendication 5, **caractérisé en ce que** le dispositif de commande (5) est conçu afin d'actionner l'élément de fermeture (3) en présence du signal de flux après une comparaison avec un état de flux prédéfini pour le point de raccordement (20A, 20B) ou de l'ouvrir au moins partiellement.

7. Dispositif de traitement extracorporel du sang (1) selon la revendication 5 ou 6, **caractérisé en ce que** le dispositif de commande (5) est conçu afin de régler, en présence du signal de flux, un débit pour le point de raccordement (20A, 20B), sur la base d'une comparaison d'un débit présent avec un débit prédéfini pour le point de raccordement (20A, 20B) et/ou sur la base d'une entrée utilisateur (64).

8. Dispositif de traitement extracorporel du sang (1) selon l'une quelconque des revendications précédentes, **caractérisé en ce que** le dispositif de commande (5) est conçu afin d'émettre un signal d'alarme (54), de préférence un signal d'alarme optique et/ou acoustique, en présence d'un signal de flux, de préférence sur la base d'une comparaison d'une entrée utilisateur avec un état de flux prédéfini pour le point de raccordement (20).

9. Dispositif de traitement extracorporel du sang (1) selon l'une quelconque des revendications précédentes, **caractérisé en ce que** la détection d'un flux de substance comprend la détection d'un élément de couplage (7) au niveau du point de raccordement (20), dans lequel la détection comprend de préférence la lecture d'un indicateur fixé sur l'élément de couplage (7) et/ou un circuit mécanique, un circuit électromécanique ou électromagnétique.

10. Dispositif de traitement extracorporel du sang (1) selon l'une quelconque des revendications précédentes, **caractérisé en ce que** le système de fluide (2) comprend un article à usage unique (8) et le point de raccordement (20A, 20B) est agencé au niveau de l'article à usage unique (8), dans lequel le un article à usage unique (8) comprend de préférence un récipient, un réservoir, un tuyau, un filtre, un filtre de dialyse, une unité d'adsorption, une cassette et/ou un cathéter.

11. Procédé de surveillance d'un point de raccordement (20A, 20B) d'un dispositif de traitement extracorporel du sang (1), de préférence un dispositif d'hémodialyse, dispositif d'hémofiltration ou dispositif d'hémodiafiltration, dans lequel le dispositif de traitement extracorporel du sang (1) présente au moins un point de raccordement (20A, 20B) pour l'ajout de substances dans un système de fluide (2) du dispositif de traitement extracorporel du sang (1), présentant les étapes suivantes :
- détection de la présence d'un flux de substance au niveau du point de raccordement (20A, 20B) ; et
- émission d'un signal (50) en présence d'un flux de substance, dans lequel le signal (50) indique une utilisation du point de raccordement (20A, 20B).

12. Procédé selon la revendication 11, **caractérisé en ce que** au moins deux points de raccordement (20A, 20B) sont prévus et la présence d'un flux de substance est associé à un des points de raccordement, dans lequel le signal (50) émis correspond au point de raccordement associé (20A, 20B).

13. Procédé selon la revendication 11 ou 12, **caractérisé en ce que** le signal (50) est émis sur la base d'une comparaison du flux de substance détecté avec un état de flux prédéfini pour le point de raccordement (20A, 20B) respectif.

14. Procédé selon l'une quelconque des revendications 11 à 13, **caractérisé en ce que** le signal comprend l'affichage d'une indication avec une invite utilisateur sur un écran et dans lequel un élément de fermeture prévu au niveau du point de raccordement est actionné sur la base d'une entrée utilisateur ou est ouvert au moins partiellement et/ou dans lequel un autre signal est émis sur la base d'une entrée utilisateur.

15. Procédé selon l'une quelconque des revendications 11 à 14, **caractérisé en ce que** la détection d'un flux de substance comprend la détection d'un élément de couplage au niveau du point de raccordement (20A, 20B), dans lequel la détection comprend de préférence la lecture d'un indicateur fixé au niveau de l'élément de couplage et/ou un circuit mécanique, un circuit électromécanique ou électromagnétique.
